# EUROPEAN PATENT SPECIFICATION

(11) **EP 2 394 173 B1**
(45) Date of publication and mention of the grant of the patent: **25.03.2015**
(21) Application number: 10703235.1
(22) Date of filing: 05.02.2010
(51) Int. Cl.: G01N 33/68, C07K 16/06, C07K 1/00, C07K 1/14, C07K 1/16, C07K 1/22

(54) **IMMUNOGLOBULIN GLYCOSYLATION PATTERN ANALYSIS**
IMMUNGLOBULIN-GLYCOSYLATIONSMUSTERANALYSE
ANALYSE DE MOTIF DE GLYCOSYLATION IMMUNOGLOBULINE

(30) Priority: 09.02.2009 EP 09001757
(43) Date of publication of application: 14.12.2011
(73) Proprietor: Roche Glycart AG, 8952 Schlieren-Zuerich (CH)
(72) Inventor: KOLL, Hans, 85247 Schwabhausen (DE); REGULA, Joerg, Thomas, 81377 Muenchen (DE); SONDERMANN, Peter, 82131 Stockdorf (DE); ZECK, Anne, 72768 Reutlingen (DE)
(74) Representative: Burger, Alexander
(86) International application number: PCT/EP2010/000710
(87) International publication number: WO 2010/089126

(56) References cited:
- LIM ET AL: "Glycosylation profiling of a therapeutic recombinant monoclonal antibody with two N-linked glycosylation sites using liquid chromatography coupled to a hybrid quadrupole time-of-flight mass spectrometer" ANALYTICAL BIOCHEMISTRY, ACADEMIC PRESS INC, NEW YORK, vol. 375, no. 2, 9 January 2008 (2008-01-09), pages 163-172, XP022519950 ISSN: 0003-2697
- NANDAKUMAR ET AL: "Therapeutic cleavage of IgG: new avenues for treating inflammation" TRENDS IN IMMUNOLOGY, ELSEVIER, RAHWAY, NJ, US, vol. 29, no. 4, 6 March 2008 (2008-03-06), pages 173-178, XP022575981 ISSN: 1471-4906
- SODERBERG J J ET AL: "The streptococcal protease IdeS modulates bacterial IgGFc binding and generates 1/2Fc fragments with the ability to prime polymorphonuclear leucocytes" MOLECULAR IMMUNOLOGY, PERGAMON, GB, vol. 45, no. 12, 1 July 2008 (2008-07-01), pages 3347-3353, XP022709825 ISSN: 0161-5890 [retrieved on 2008-06-03]
- BENNETT K L ET AL: "Monitoring papain digestion of a monoclonal antibody by electrospray ionization mass spectrometry" ANALYTICAL BIOCHEMISTRY, ACADEMIC PRESS INC, NEW YORK, vol. 245, no. 1, 1 January 1997 (1997-01-01), pages 17-27, XP002297987 ISSN: 0003-2697

### SEQUENCE LISTING

<110> F. Hoffmann-La Roche AG
<120> Immunoglobulin Glycosylation Pattern Analysis
<130> 25905 WO
<150> EP09001757.5
   <151> 2009-02-09
<160> 5
<170> PatentIn version 3.5
<210> 1
   <211> 339
   <212> PRT
   <213> Streptococcus pyogenes
<400> 1
   Met Arg Lys Arg Cys Tyr Ser Thr Ser Ala Ala Val Leu Ala Ala Val
   1               5                   10                  15
   Thr Leu Phe Val Leu Ser Val Asp Arg Gly Val Ile Ala Asp Ser Phe
               20                  25                  30
   Ser Ala Asn Gln Glu Ile Arg Tyr Ser Glu Val Thr Pro Tyr His Val
           35                  40                  45
   Thr Ser Val Trp Thr Lys Gly Val Thr Pro Pro Ala Asn Phe Thr Gln
       50                  55                  60
   Gly Glu Asp Val Phe His Ala Pro Tyr Val Ala Asn Gln Gly Trp Tyr
   65                  70                  75                  80
   Asp Ile Thr Lys Thr Phe Asn Gly Lys Asp Asp Leu Leu Cys Gly Ala
                   85                  90                  95
   Ala Thr Ala Gly Asn Met Leu His Trp Trp Phe Asp Gln Asn Lys Asp
               100                 105                 110
   Gln Ile Lys Arg Tyr Leu Glu Glu His Pro Glu Lys Gln Lys Ile Asn
           115                 120                 125
   Phe Asn Gly Glu Gln Met Phe Asp Val Lys Glu Ala Ile Asp Thr Lys
   130 135 140
   Asn His Gln Leu Asp Ser Lys Leu Phe Glu Tyr Phe Lys Glu Lys Ala
   145 150 155 160
   Phe Pro Tyr Leu Ser Thr Lys His Leu Gly Val Phe Pro Asp His Val
   165 170 175
   Ile Asp Met Phe Ile Asn Gly Tyr Arg Leu Ser Leu Thr Asn His Gly
   180 185 190
   Pro Thr Pro Val Lys Glu Gly Ser Lys Asp Pro Arg Gly Gly Ile Phe
   195 200 205
   Asp Ala Val Phe Thr Arg Gly Asp Gln Ser Lys Leu Leu Thr Ser Arg
   210 215 220
   His Asp Phe Lys Glu Lys Asn Leu Lys Glu Ile Ser Asp Leu Ile Lys
   225 230 235 240
   Lys Glu Leu Thr Glu Gly Lys Ala Leu Gly Leu Ser His Thr Tyr Ala
   245 250 255
   Asn Val Arg Ile Asn His Val Ile Asn Leu Trp Gly Ala Asp Phe Asp
   260 265 270
   Ser Asn Gly Asn Leu Lys Ala Ile Tyr Val Thr Asp Ser Asp Ser Asn
   275 280 285
   Ala Ser Ile Gly Met Lys Lys Tyr Phe Val Gly Val Asn Ser Ala Gly
   290 295 300
   Lys Val Ala Ile Ser Ala Lys Glu Ile Lys Glu Asp Asn Ile Gly Ala
   305 310 315 320
   Gln Val Leu Gly Leu Phe Thr Leu Ser Thr Gly Gln Asp Ser Trp Asn
   325 330 335
   Gln Thr Asn
<210> 2
   <211> 224
   <212> PRT
   <213> Mus musculus
<400> 2
   Ala Lys Thr Thr Ala Pro Ser Val Tyr Pro Leu Ala Pro Val Cys Gly
   1 5 10 15
   Asp Thr Thr Gly Ser Ser Val Thr Leu Gly Cys Leu Val Lys Gly Tyr
   20 25 30
   Phe Pro Glu Pro Val Thr Leu Thr Trp Asn Ser Gly Ser Leu Ser Ser
   35 40 45
   Gly Val His Thr Phe Pro Ala Val Leu Gln Ser Asp Leu Tyr Thr Leu
   50 55 60
   Ser Ser Ser Val Thr Val Thr Ser Ser Thr Trp Pro Ser Gln Ser Ile
   65 70 75 80
   Thr Cys Asn Val Ala His Pro Ala Ser Ser Thr Lys Val Asp Lys Lys
   85 90 95
   Ile Glu Pro Arg Gly Pro Thr Ile Lys Pro Cys Pro Pro Cys Lys Cys
   100 105 110
   Pro Ala Pro Asn Leu Leu Gly Gly Pro Ser Val Phe Ile Phe Pro Pro
   115 120 125
   Lys Ile Lys Asp Val Leu Met Ile Ser Leu Ser Pro Ile Val Thr Cys
   130 135 140
   Val Val Val Asp Val Ser Glu Asp Asp Pro Asp Val Gln Ile Ser Trp
   145 150 155 160
   Phe Val Asn Asn Val Glu Val His Thr Ala Gln Thr Gln Thr His Arg
   165 170 175
   Glu Asp Tyr Asn Ser Thr Leu Arg Val Val Ser Ala Leu Pro Ile Gln
   180 185 190
   His Gln Asp Trp Met Ser Gly Lys Glu Phe Lys Cys Lys Val Asn Asn
   195 200 205
   Lys Asp Leu Pro Ala Pro Ile Glu Arg Thr Ile Ser Lys Pro Lys Gly
   210 215 220
<210> 3
   <211> 223
   <212> PRT
   <213> Mus musculus
<400> 3
   Thr Thr Thr Ala Pro Ser Val Tyr Pro Leu Val Pro Gly Cys Ser Asp
   1               5                   10                  15
   Thr Ser Gly Ser Ser Val Thr Leu Gly Cys Leu Val Lys Gly Tyr Phe
               20                  25                  30
   Pro Glu Pro Val Thr Val Lys Trp Asn Tyr Gly Ala Leu Ser Ser Gly
           35                  40                  45
   Val Arg Thr Val Ser Ser Val Leu Gln Ser Gly Phe Tyr Ser Leu Ser
       50                  55                  60
   Ser Leu Val Thr Val Pro Ser Ser Thr Trp Pro Ser Gln Thr Val Ile
   65                  70                  75                  80
   Cys Asn Val Ala His Pro Ala Ser Lys Thr Glu Leu Ile Lys Arg Ile
                   85                  90                  95
   Glu Pro Arg Ile Pro Lys Pro Ser Thr Pro Pro Gly Ser Ser Cys Pro
               100                 105                 110
   Pro Gly Asn Ile Leu Gly Gly Pro Ser Val Phe Ile Phe Pro Pro Lys
           115                 120                 125
   Pro Lys Asp Ala Leu Met Ile Ser Leu Thr Pro Lys Val Thr Cys Val
       130                 135                 140
   Val Val Asp Val Ser Glu Asp Asp Pro Asp Val His Val Ser Trp Phe
   145                 150                 155                 160
   Val Asp Asn Lys Glu Val His Thr Ala Trp Thr Gln Pro Arg Glu Ala
   165 170 175
   Gln Tyr Asn Ser Thr Phe Arg Val Val Ser Ala Leu Pro Ile Gln His
   180 185 190
   Gln Asp Trp Met Arg Gly Lys Glu Phe Lys Cys Lys Val Asn Asn Lys
   195 200 205
   Ala Leu Pro Ala Pro Ile Glu Arg Thr Ile Ser Lys Pro Lys Gly
   210 215 220
<210> 4
   <211> 330
   <212> PRT
   <213> Homo sapiens
<400> 4
<210> 5
   <211> 327
   <212> PRT
   <213> Homo sapiens
<400> 5
   Ala Ser Thr Lys Gly Pro Ser Val Phe Pro Leu Ala Pro Cys Ser Arg
   1               5                   10                  15
   Ser Thr Ser Glu Ser Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Lys Thr
   65                  70                  75                  80
   Tyr Thr Cys Asn Val Asp His Lys Pro Ser Asn Thr Lys Val Asp Lys
                   85                  90                  95
   Arg Val Glu Ser Lys Tyr Gly Pro Pro Cys Pro Ser Cys Pro Ala Pro
               100                 105                 110
   Glu Phe Leu Gly Gly Pro Ser Val Phe Leu Phe Pro Pro Lys Pro Lys
           115                 120                 125
   Asp Thr Leu Met Ile Ser Arg Thr Pro Glu Val Thr Cys Val Val Val
       130                 135                 140
   Asp Val Ser Gln Glu Asp Pro Glu Val Gln Phe Asn Trp Tyr Val Asp
   145                 150                 155                 160
   Gly Val Glu Val His Asn Ala Lys Thr Lys Pro Arg Glu Glu Gln Phe
                   165                 170                 175
   Asn Ser Thr Tyr Arg Val Val Ser Val Leu Thr Val Leu His Gln Asp
               180                 185                 190
   Trp Leu Asn Gly Lys Glu Tyr Lys Cys Lys Val Ser Asn Lys Gly Leu
           195                 200                 205
   Pro Ser Ser Ile Glu Lys Thr Ile Ser Lys Ala Lys Gly Gln Pro Arg
       210                 215                 220
   Glu Pro Gln Val Tyr Thr Leu Pro Pro Ser Gln Glu Glu Met Thr Lys
   225                 230                 235                 240
   Asn Gln Val Ser Leu Thr Cys Leu Val Lys Gly Phe Tyr Pro Ser Asp
                   245                 250                 255
   Ile Ala Val Glu Trp Glu Ser Asn Gly Gln Pro Glu Asn Asn Tyr Lys
               260                 265                 270
   Thr Thr Pro Pro Val Leu Asp Ser Asp Gly Ser Phe Phe Leu Tyr Ser
           275                 280                 285
   Arg Leu Thr Val Asp Lys Ser Arg Trp Gln Glu Gly Asn Val Phe Ser
       290                 295                 300
   Cys Ser Val Met His Glu Ala Leu His Asn His Tyr Thr Gln Lys Ser
   305                 310                 315                 320
   Leu Ser Leu Ser Leu Gly Lys
                   325

## Description

The current invention is directed to a method for the determination of the glycosylation pattern of an immunoglobulin Fc-fragment. Also is presented a method for the determination of the glycosylation pattern of a Fab-glycosylated immunoglobulin as well as a method of producing an immunoglobulin Fab₂-fragment.

### Background of the Invention

The pharmaceutical industry has been very successful in recent years with products based among others on enzymes, antibodies and cytokines such as e.g. erythropoietin, interferons, plasminogen activator etc. and the worldwide demand for protein therapeutic agents increases every year. Therapeutic monoclonal antibodies (mAbs, monoclonal antibodies) are an important group within the protein therapeutics. They are referred to as monoclonal because, in contrast to polyclonal antibodies, they are secreted by immune cells (cell clones) which are derived from a single antibody-forming cell. A characteristic of monoclonal antibodies is that they are each only directed against one epitope of an immunogenic substance and can therefore be used very specifically in the treatment of diseases. Examples of protein therapeutics are the monoclonal antibodies trastuzumab (commercial name: Herceptin), daclizumab (commercial name: Zenapax) and rituximab (commercial name: MabThera) manufactured by Roche which have been used successfully for the treatment of among others breast cancer (trastuzumab), for organ rejection (daclizumab) and non-Hodgkin lymphoma (rituximab).

Therapeutic monoclonal antibodies are obtained by means of complex biotechnological processes. Degradation products may be formed during their production, formulation and storage which are often due to processes like oxidation and deamidation as well as proteolytic cleavages (Yan, B., et al., J. Chromatogr. A 1164 (2007) 153-161). The quality, i.e. the purity, integrity, aggregation state and the glycosylation pattern, of a biopharmaceutical product is of importance in addition to its action.

The cysteine endoprotease IdeS (Immunoglobulin degrading enzyme S) from the human pathogen Streptococcus pyogenes, which is also referred to as Mac-1 or sib-38, is a cysteine protease that specifically cleaves the heavy chain of antibodies of the immunoglobulin G type (IgG). IgG is hitherto the only known substrate of IdeS (Vincents, B., et al., Biochem. 43 (2004) 15540-15549). IdeS consists of 339 amino acids including a signal peptide comprising 29 amino acids (von Pawel-Rammingen, U., et al., EMBO J. 21 (2002) 1607-1615). IdeS cleaves human IgG subclasses IgG1, IgG3 and IgG4 between the amino acids 236 and 237 (Gly-Gly) which are contained in the recognition sequence (LL)GGP. Human IgG2 is cleaved between the amino acids alanine and glycine in the recognition motif PVAGP. Murine antibodies of the IgG2a, IgG2b and IgG3 type as well as rabbit IgG (LLGGPS) are also cleaved (see e.g. Vincents, B., et al., Biochem. 43 (2004) 15540-15549 ; Wenig, K., Proc. Natl. Acad. Sci. USA 101 (2004) 17371-17376).

Hess et al. (Hess, J.K., et al., J. Microbiol. Meth. 70 (2007) 284-291) report a mass spectroscopic method for determining the enzymatic activity of IdeS with the aid of SELDI-TOF mass spectrometry. A polypeptide which was isolated from S. pyogenes and has an IgG cysteine protease activity is reported in the US 2007/0237784. A method for forming Fc or Fab fragments of antibodies is reported in the EP 1 458 861 A. IdeS protease from group A streptococci is reported in WO 2006/131347.

The glycosylation profile, e.g. of a polypeptide, is an important characteristic for many recombinantly produced therapeutic polypeptides. Glycosylated polypeptides, also termed glycoproteins, mediate many essential functions in eukaryotic organisms, e.g. humans, and some prokaryotes, including catalysis, signaling, cell-cell communication, activities of the immune system, as well as molecular recognition and association. They make up the majority of non-cytosolic proteins in eukaryotic organisms (Lis, H., et al., Eur. J. Biochem. 218 (1993) 1-27). The formation/attachment of oligosaccharides to a protein is a co- and posttranslational modification and, thus, is not genetically controlled. The biosynthesis of oligosaccharides is a multistep process involving several enzymes, which compete with each other for the substrate. Consequently, glycosylated polypeptides comprise a microheterogeneous array of oligosaccharides, giving rise to a set of different glycoforms containing the same amino acid backbone.

The covalently bound oligosaccharides do influence physical stability, folding, resistance to protease attack, interactions with the immune system, bioactivity, and pharmacokinetics of the respective polypeptide. Moreover some glycoforms can be antigenic, prompting regulatory agencies to require analysis of the oligosaccharide structures of recombinant glycosylated polypeptides (see e.g. Paulson, J.C., Trends Biochem. Sci. 14 (1989) 272-276; Jenkins, N., et al., Nature Biotechnol. 14 (1998) 975-981). Terminal sialylation of glycosylated polypeptides for example has been reported to increase serum-half life, and glycosylated polypeptides containing oligosaccharide structures with terminal galactose residues show increased clearance from circulation (Smith, P.L., et al., J. Biol. Chem. 268 (1993) 795-802).

Immunoglobulins differ from other recombinant polypeptides in their glycosylation pattern. Immunoglobulin G (IgG) for example is a symmetrical, multifunctional glycosylated polypeptide of an approximate molecular mass of 150 kDa consisting of two identical Fab-fragments responsible for antigen binding and the Fc-fragment for effector functions. Glycosylation tends to be highly conserved in IgG molecules at Asn-297, which is buried between the C_{H}2 domains of the Fc heavy chain, forming extensive contacts with the amino acid residues within C_{H}2 (Sutton and Phillips, Biochem. Soc. Trans. 11 (1983) 130-132). The Asn-297 linked oligosaccharide structures are heterogeneously processed, such that an IgG exist in multiple glycoforms. Variations exist in the site occupancy of the Asn-297 residue (macroheterogeniety) or by variation in the oligosaccharide structure at the glycosylation site (microheterogeniety), see for example Jenkins, N., et al., Nature Biotechnol. 14 (1996) 975-981.

High performance anion exchange chromatography with pulsed amperometric detection (HPAEC) and matrix-assisted laser desorption ionization time-of-flight mass spectrometry (MALDI-TOF MS) have been used to analyze the carbohydrate moieties of glycosylated polypeptides (see e.g. Fukuda, M., (ed.) Glycobiology: A Practical Approach, IRL Press, Oxford; Morelle, W. and Michalsky, J.C., Curr. Pharmaceut. Design 11 (2005) 2615-2645). Hoffstetter-Kuhn et al. (Electrophoresis 17 (1996) 418-422) used capillary electrophoresis and MALDI-TOF MS analysis to profile the oligosaccharide-mediated heterogeneity of a monoclonal antibody after deglycosylation of the antibody with N-glycosidase F (PNGase F).

Given the importance of glycosylation on functional properties of recombinant glycosylated polypeptides and the necessity of a well-defined and consistent product production process, an on-line or ad-line analysis of the glycosylation pattern of recombinantly produced glycosylated polypeptides during the fermentation process is highly desirable. Papac et al. (Glycobiol. 8 (1998) 445-454) reported a method containing the immobilization of glycosylated polypeptides on a polyvinylidene difluoride membrane, the enzymatic digestion and MALDI-TOF MS analysis of the glycosylation profile. The analysis and the molecular characterization of recombinantly produced mAbs, including several chromatography steps, is reported in Bailey, M., et al., J. Chromat. 826 (2005) 177-187.

Immunoglobulins produced by mammalian cells contain 2-3 % by mass carbohydrates (Taniguchi, T., et al., Biochem. 24 (1985) 5551-5557). This is equivalent e.g. in an immunoglobulin of class G (IgG) to 2.3 sugar residues in an IgG of mouse origin (Mizuochi, T., et al., Arch. Biochem. Biophys. 257 (1987) 387-394) and to 2.8 sugar residues in an IgG of human origin (Parekh, R.B., et al., Nature 316 (1985) 452-457), whereof generally two are located in the Fc-region at Asn-297 and the remaining in the variable region (Saba, J.A., et al., Anal. Biochem. 305 (2002) 16-31).

For an immunoglobulin of class G (IgG), for example, different N-glycosylation sites at which oligosaccharides are or can be bound to the amino acid backbone are known. In the Fc-region of an IgG oligosaccharide residues can be introduced via N-glycosylation at amino acid residue 297, which is an asparagine (denoted as Asn-297). Youings et al. have shown that further N-glycosylation site exists in 15-20 % of polyclonal IgG molecules in the Fab-region (Youings, A., et al., Biochem. J., 314 (1996) 621-630; see e.g. also Endo, T., et al., Mol. Immunol. 32 (1995) 931-940).

Due to inhomogeneous, i.e. asymmetric, oligosaccharide processing multiple glycostructure isoforms of immunoglobulins exist (Patel, T.P., et al., Biochem. J. 285 (1992) 839-845; Yu-Ip, C.C., et al., Arch. Biochem. Biophys. 308 (1994) 387-399; Lund, J., et al., Immunol. 30 (1993) 741-748). Concurrently the structure and distribution of the oligosaccharides is both highly reproducible (i.e. non-random) and site specific (Dwek, R.A., et al., J. Anat. 187 (1995) 279-292).

Some characteristics of an immunoglobulin are directly linked to the glycosylation of the Fc-region (see e.g. Dwek, R.A., et al., J. Anat. 187 (1995) 279-292; Lund, J., et al., J Immunol. 157 (1996) 4963-4969 and FASEB J. 9 (1995) 115-119; Wright, A., and Morrison, S.L., J. Immunol. 160 (1998) 3393-3402), such as for example thermal stability and solubility (West, C.M., Mol. Cell. Biochem. 72 (1986) 3-20), antigenicity (Turco, S.J., Arch. Biochem. Biophys. 205 (1980) 330-339), immunogenicity (Bradshaw, J.P., et al., Biochim. Biophys. Acta 847 (1985) 344-351; Feizi, T., and Childs, R.A., Biochem. J. 245 (1987) 1-11; Schauer, R., Adv. Exp. Med. Biol. 228 (1988) 47-72), clearance rate/circulatory half-life (Ashwell, G., and Harford, J., Ann. Rev. Biochem. 51 (1982) 531-554; McFarlane, I.G., Clin. Sci. 64 (1983) 127-135; Baenziger, J.U., Am. J. Path. 121 (1985) 382-391; Chan, V.T., and Wolf, G., Biochem. J. 247 (1987) 53-62; Wright, A., et al., Glycobiology 10 (2000) 1347-1355; Rifai, A., et al., J. Exp. Med. 191 (2000) 2171-2182; Zukier, L.S., et al., Cancer Res. 58 (1998) 3905-3908), and biological specific activity (Jefferis, R., and Lund, J., in Antibody Engineering, ed. by Capra, J.D., Chem. Immunol. Basel, Karger, 65 (1997) 111-128).

Glycosylation profiling of a therapeutic recombinant monoclonal antibody with two N-linked glycosylation sites using liquid chromatography coupled to a hybrid quadrupole time-of-flight mass spectrometer is reported in Lim, A., et al., Anal. Biochem. 375 (2008) 163-172. Nandakumar, K.S. and Holmdahl, R., Trends. Immunol. 29 (2008) 173-178 report the possibility of cleaving or modifying IgG *in vivo* by injection of enzymes. The streptococcal protease Ides modulates bacterial IgGFc binding and generates 1/2Fc fragments with the ability to prime polymorphonuclear leucocytes is reported by Söderberg, J.J. and von Pawel-Rammingen, U., Mol. Immunol. 45 (2008) 3347-3353. Bennet, K.L., et al., Anal. Biochem. 245 (1997) 17-27 report the monitoring papain digestion of a monoclonal antibody by electrospray ionization mass spectrometry.

### Summary of the Invention

One aspect of the current invention is a method for the determination of the glycosylation pattern or the site-specific glycosylation pattern of a human immunoglobulin of the subclass IgG1 (huIgG1) or IgG2 (huIgG2) or IgG4 (huIgG4) comprising the following steps:
a) cleaving the immunoglobulin into fragments by enzymatic digestion with the enzyme IdeS and treating the immunoglobulin fragments with carboxypeptidase B,
ab) treating the digested immunoglobulin of step a) with formic acid and TCEP
b) separating the fragments of the immunoglobulin obtained in a) by reversed phase high performance liquid chromatography,
c) subjecting the separated fragments of the immunoglobulin obtained in b) to a mass spectrometric analysis wherein the mass spectrometric analysis is performed in the absence of trifluoro acetic acid, and
d) determining the glycosylation pattern of the immunoglobulin from the mass spectrometric data obtained in c).

In one embodiment comprises the method according to the invention the following steps b) and c):
b) desalting the fragments of the immunoglobulin by a size exclusion chromatography,
c) directly applying the desalted fragments to mass spectrometric analysis.

### Detailed Description of the Invention

The current invention is directed to a method for the determination of the glycosylation pattern of a human immunoglobulin of the subclass IgG1 (huIgG1) or IgG2 (huIgG2) or IgG4 (huIgG4) by using the enzyme IdeS and mass spectrometric analysis.

It has been found that the use of IdeS provides for a highly reproducible cleavage even under non-reducing conditions. Furthermore the addition of TFA (trifluoro acetic acid) can be omitted for mass spectrometric analysis, i.e. the mass spectrometric analysis is performed in the absence of trifluoro acetic acid. Furthermore the use of IdeS is not limited, e.g. it can be used to digest an immunoglobulin in solution.

The ESI-MS technique is generally performed at the level of intact immunoglobulin or immunoglobulin heavy chain and provides for a lower mass resolution for large proteins. The ESI-MS analysis of papain digested immunoglobulins is limited due to the low specificity of the enzyme and the requirement of cysteine for its activation, which causes the formation of side-product, i.e. artifacts that interfere in the data analysis, e.g. by reduction reactions. In the peptide-map technique a relatively complicated sample preparation has to be performed and the quantitative analysis is difficult due to among other things the formation of salt adducts.

Generally all chromatographic methods require substance labeling, the co-elution of certain glycans cannot be avoided. Additionally it is not possible to distinguish the glycosylation pattern of immunoglobulins glycosylated in the Fc-fragment as well as in the Fab-fragment.

A "polypeptide" is a polymer consisting of amino acids which are linked together by peptide bonds. It can either be produced enzymatically or synthetically. Polypeptides containing less than 20 amino acids are also referred to as "peptides". A "protein" is a macromolecule which contains two or more polypeptides or it is a polypeptide which is composed of more than 100 amino acids. A polypeptide can also contain non-peptidic components such as e.g. carbohydrates. The non-peptidic modifications are introduced by the cell which expresses the polypeptide and therefore depend on the cell type. In this application polypeptides are defined by their amino acid sequence. Modifications such as carbohydrates are not explicitly described but may always be present.

The terms "antibody" and "immunoglobulin" that are used synonymously within this application denote a molecule which contains at least two light polypeptide chains (light chain, LC) and two heavy polypeptide chains (heavy chain, HC). Each of the light and heavy chains comprises a variable region (normally the amino terminus of the chain) which contains binding domains for binding of an antigen. Each of the heavy and light chains comprises a constant region (normally the carboxy terminus of the chain) which mediates the binding of the antibody to different receptors. A light chain is normally composed of a variable domain V_{L} and a constant domain C_{L}. A heavy chain is normally composed of a variable domain V_{H} and a constant region which in turn comprises the domains C_{H}1, hinge, C_{H}2, C_{H}3 and optionally C_{H}4. Antibodies may occur in numerous forms e.g. as Fv, Fab, and F(ab)₂ as well as single chains (scFv) (e.g. Huston, J.S., et al., Proc. Natl. Acad. Sci. USA 85 (1988) 5879-5883; Bird, R.E., et al., Science 242 (1988) 423-426; and Hood, L. E., et al., Immunology, Benjamin N.Y., 2nd Edition (1984) and Hunkapiller, T. and Hood, L., Nature 323 (1986) 15-16). Immunoglobulins are divided into classes depending on the amino acid sequence of the constant region of the heavy chain: IgA, IgD, IgE, IgG and IgM. Some of these classes are further subdivided into subclasses (isotypes) e.g. IgG into IgG1, IgG2, IgG3 and IgG4 or IgA into IgA1 and IgA2. The constant regions of the heavy chains are referred to as α (IgA), δ (IgD), ε (IgE), γ (IgG) and µ (IgM) depending on the class to which the antibody belongs.

General chromatographic methods are known to a person skilled in the art e.g. Chromatography, 5th edition, Part A: Fundamentals and Techniques, Heftmann, E. (ed.), Elsevier Science Publishing Company, New York, (1992); Advanced Chromatographic and Electromigration Methods in Biosciences, Deyl, Z. (ed.), Elsevier Science BV, Amsterdam, The Netherlands, (1998); Chromatography Today, Poole, D.F. and Poole, S.K., Elsevier Science Publishing Company, New York, (1991); Scopes, R.K., Protein Purification: Principles and Practice (1982); Sambrook, J., et al. (ed.), Molecular Cloning: A Laboratory Manual, Second Edition, Cold Spring Harbor Laboratory Press, Cold Spring Harbor, N.Y., 1989; or Current Protocols in Molecular Biology, Ausubel, F.M., et al. (eds.), John Wiley & Sons, Inc., New York.

It has been found that the immunoglobulin degrading enzyme IdeS, a cysteine protease cleaving IgGs highly specific at the GP(S) motif can be used in combination with LC-MS (liquid chromatography combined with mass spectrometry) for detailed site-specific glycosylation pattern characterization of immunoglobulins.

It has been found that in a two hour digest procedure IdeS cleaves antibodies resulting in two HC-Fc-fragment, also termed HC-Fc-fragment (comprising the C-terminal parts of the immunoglobulin heavy chains) and the Fab₂-fragment, also termed Fab₂-fragment (comprising the N-terminal parts of the immunoglobulin heavy chains and the immunoglobulin light chains). This method is especially suitable for the analysis of the glycosylation pattern of immunoglobulins glycosylated in the Fc-fragment or glycosylated in the Fc-fragment and in the Fab₂-fragment. For the analysis of a secondary glycosylation site the IdeS digest can be reduced. It is also possible to analyze degradation products with this method. After the enzymatic treatment the sample is subjected to LC-MS analysis comprising a reversed phase high performance liquid chromatography (RP-HPLC) and an online MS-analysis on a QTOF instrument (quadrupole time-of-flight mass spectrometer). The method according to the invention can be used e.g. for batch characterization, for monitoring down stream processing steps as well as for monitoring fermentation processes even without sample clean-up.

The GP(S) motif in the N-terminal region of the C_{H}2 domain of immunoglobulins is essential for digestion with IdeS. As used herein the term HC-Fc-fragment refers to the C-terminal fragment of an immunoglobulin heavy chain obtained by IdeS digestion and the term Fab₂-fragment refers to the N-terminal fragment of an immunoglobulin including the complete light chains obtained by IdeS digestion. Therefore, in one embodiment the method according to the invention is for characterizing chimeric or humanized IgG, i.e. for human or humanized immunoglobulins of the subclass IgG1, IgG2 or IgG4, or a variant thereof. In one embodiment the digest is performed at slightly basic pH for two hours. For the analysis of the glycosylation profile in the Fab₂-fragment an additional reduction step under acidic conditions using TCEP for half an hour is performed in one embodiment. The sample is afterwards subjected to the LC-MS analysis as described below and in the examples.

It has been found that in contrast to the glycosylation pattern analysis at the level of intact immunoglobulin heavy chain or even intact immunoglobulin the method has the advantage of higher mass accuracy, resolution and sensitivity. Compared to other enzymes like papain or limited LysC digestion IdeS shows the advantage of having a unique cleavage site and, therefore, being highly specific and cleaving the different subclasses with similar efficiency. No risk of excessive cleavage exists and no cysteine is required for activation of the enzyme. For analysis of degradation products the IdeS digest is reduced to yield three antibody fragments: the full length immunoglobulin light chain, two heavy chain Fab-fragments (HC Fab-fragment) and the heavy chain Fc-fragment (HC Fc-fragment). The three species can be separated by chromatography and analyzed individually. This approach is an easy and fast way to determine whether degradations such as oxidation, deamidation or fragmentation are present/occurred in the Fc-fragment or in the Fab-fragment of an immunoglobulin. Certain degradation reactions, such as e.g. oxidation, lead to a slight shift in the retention time and can therefore be used for fast monitoring of samples from formulation and stability studies by using UV absorption detection, which is also an aspect of the current invention. In one embodiment the methods according to the invention comprise a de-salting step using a size exclusion chromatography with direct mass spectrometric analysis of the desalted molecule mixture.

### Specific cleavage of immunoglobulins

Monoclonal immunoglobulins are very large proteins and, furthermore, are extremely heterogeneous (microheterogenicity) due to the glycostructures of their heavy chains. In order to examine the glycosylation pattern of immunoglobulins for the formation of degradation products and/or for modifications it is expedient to cleave them into smaller fragments before the glycostructure analysis.

### Cleavage of disulfide bridges

All disulfide bridges occurring in an immunoglobulin molecule can be cleaved by reduction. The free heavy and light chains of an immunoglobulin are obtained during the reduction. Tris-(2-carboxyethyl)-phosphine (TCEP) is a reducing agent that is often used because all disulfide bridges of an immunoglobulin are completely cleaved within a short period and the reduction occurs in the entire pH range (see e.g. Hau, J.C. and Hau, C.Y., Anal. Biochem. 220 (1994) 5-10). In one embodiment the pH range is of from pH 1.5 to pH 8.5. Dithiothreitol (DTT) is also characterized by a rapid cleavage of disulfide bridges. However, the DTT reduction in an acidic environment only proceeds very poorly. A denaturing step is usually necessary to complete the dissociation of the heavy and light chain. The denaturation additionally makes the disulfide groups more accessible. The denaturation in one embodiment can for example be carried out with the aid of guanidine/HCl or formic acid.

### Enzymatic cleavage

Papain, a cysteine protease, cleaves peptide bonds relatively unspecifically after arginine (R), lysine (K), glutamic acid (E), histidine (H), glycine (G) and tyrosine (Y). If the incubation period is sufficiently or too long, the papain digestion leads to total hydrolysis of the immunoglobulin. However, immunoglobulins can be cleaved relatively selectively in their hinge region by a limited proteolysis (Lottspeich, F. and Engels, J.W., Bioanalytik Spektrum Akademischer Verlag, Munich, 2nd Edition (2006) 201-214). The cleavage occurs on the N-terminal side of the disulfide bridges which connect the two heavy chains. The disulfide bridges are retained in this process so that three fragments (2 Fab fragments, 1 Fc fragment) are obtained. The two N-terminal fragments are referred to as antigen-binding fragments (Fab-fragment, Fab, antigen-binding fragment), the C-terminal fragment is referred to as the crystalline fragment (Fc-fragment, Fc, crystallizing fragment). Each Fab-fragment is composed of a complete light chain and the amino-terminal half of the heavy chain. The Fc-fragment is composed of the two carboxy-terminal halves of the heavy chains which are still linked together by a disulfide bridge.

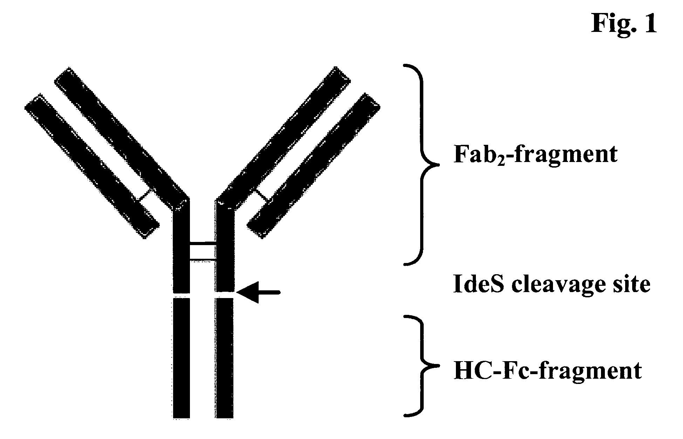

IdeS (immunoglobulin G-degrading enzyme of S. pyogenes) is a cellular cysteine protease which can be isolated from the pathogenic bacterium Streptococcus pyogenes. This enzyme cleaves human IgG with high specificity directly before the sequence GP(SVFLFP), i.e. the GP(S) motif. This sequence is located on the C-terminal side of disulfide bridges which link the two heavy chains (HC) together. The cleavage results in the C-terminal ends of the two heavy chains (2 HC-Fc-fragment) and a Fab₂-fragment comprising the Fab-fragment of the light and heavy chain that are linked by disulfide bridges (Figure 1) (von Pawel-Rammingen, U., et al., EMBO Journal 21 (2002) 1607-1615). If the fragments obtained by IdeS cleavage of the immunoglobulin are reduced with DTT or TCEP after the digestion, the two light chains of the antibody (2 LC) and the N-terminal fragments of the heavy chains (2 HC Fab-fragment) are obtained instead of the Fab₂-fragment. The C-terminal ends of the heavy chain (HC-Fc) are not affected by the reduction.

The generally employed enzyme papain for immunoglobulin cleavage cleaves N-terminal to the hinge region. Therefore the Papain digest necessitates reduction and all three fragments of the Ab (Fc, LC and HC Fab) appear in the same m/z range in the mass spectrum.

The first aspect of the current invention is a method for the determination of the glycosylation pattern of a human or humanized immunoglobulin of the subclass IgG1 or IgG2 or IgG4 or of a variant thereof comprising the following steps:
a) cleaving said immunoglobulin into fragments by enzymatic digestion with the enzyme IdeS and treating said immunoglobulin with carboxypeptidase B,
b) treating said enzymatically cleaved immunoglobulin with formic acid and TCEP,
c) separating the enzymatically cleaved fragments of the immunoglobulin obtained by the enzymatic digestion by reversed phase high performance liquid chromatography,
d) subjecting the separated fragments of the immunoglobulin obtained in step c) to a mass spectrometric analysis wherein the mass spectrometric analysis is performed in the absence of trifluoro acetic acid, and
e) determining the glycosylation pattern of the immunoglobulin from the mass spectrometric data obtained in step d).

It has been found that with the use of the specifically cleaving enzyme IdeS instead of e.g. a limited proteolysis with LysC or papain, defined fragments of immunoglobulins can be obtained which are very well suited for the determination of the glycosylation pattern of an immunoglobulin.

The term "glycosylation pattern" as used within this application denotes the oligosaccharides as a whole which are attached to one or more specified amino acid residue in an immunoglobulin. Due to the glycosylation heterogeneity of a cell, a recombinantly produced immunoglobulin comprises not only a single, defined N- or O-linked oligosaccharide at a specified amino acid residue, but is a mixture of polypeptides each having the same amino acid sequence but comprising differently composed oligosaccharides at the specified amino acid position. Thus, the above term denotes a group of oligosaccharides that are attached to one or more specified amino acid position of a recombinantly produced immunoglobulin, i.e. the heterogeneity of the attached oligosaccharide. The term "oligosaccharide" as used within this application denotes a polymeric saccharide comprising two or more covalently linked monosaccharide units.

In the method according to the invention the immunoglobulin from which the glycosylation pattern has to be determined is in a first step enzymatically digested / cleaved with the enzyme IdeS in an HC Fc-fragment and a Fab₂-fragment. If the glycosylation pattern of a glycosylation site in the Fab₂-fragment has to be determined a reduction of the immunoglobulin disulfide bonds with a reducing agent is performed. After the enzymatic cleavage and the reduction step the solution is acidified in order to induce the dissociation of the two heavy chain Fc-fragments. The acidification is in the same step as the reduction of the immunoglobulin disulfide bonds. The obtained fragments are separated by reverse phase HPLC (RP-HPLC). For the determination of the glycosylation pattern the sample containing the dissociated heavy chain Fc-fragments and optionally the Fab₂- or Fab-fragments of the immunoglobulin is subjected to a mass spectrometric (MS) analysis. With the results of the MS analysis the glycosylation pattern of the immunoglobulin is determined. In one embodiment the MS analysis is performed in a separated step aside from the RP-HPLC (offline). In another embodiment the MS analysis is performed directly after the RP-HPLC (online), i.e. the eluent of the RP-HPLC is directly introduced into the MS equipment.

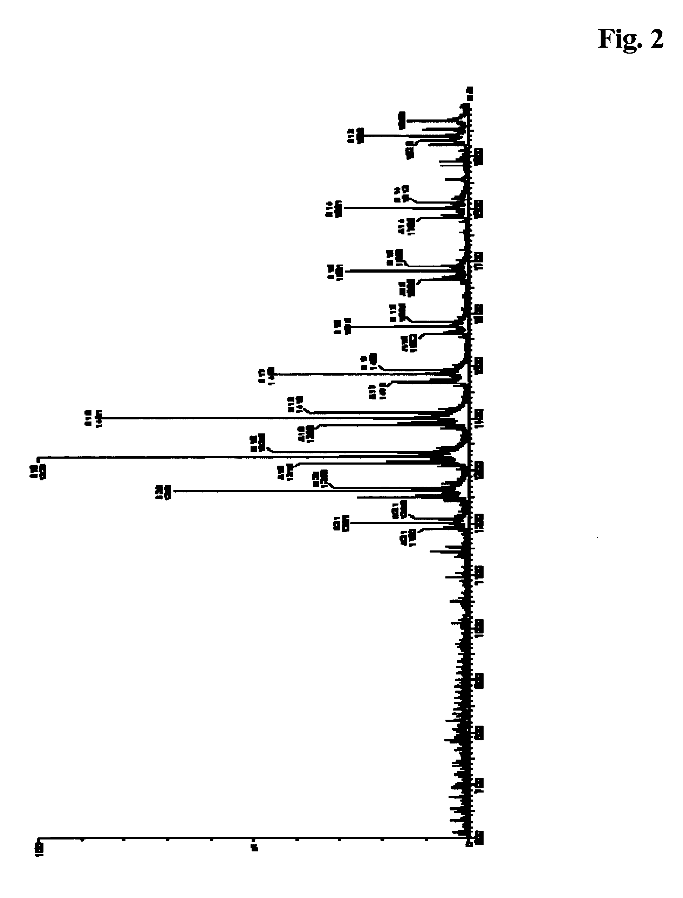

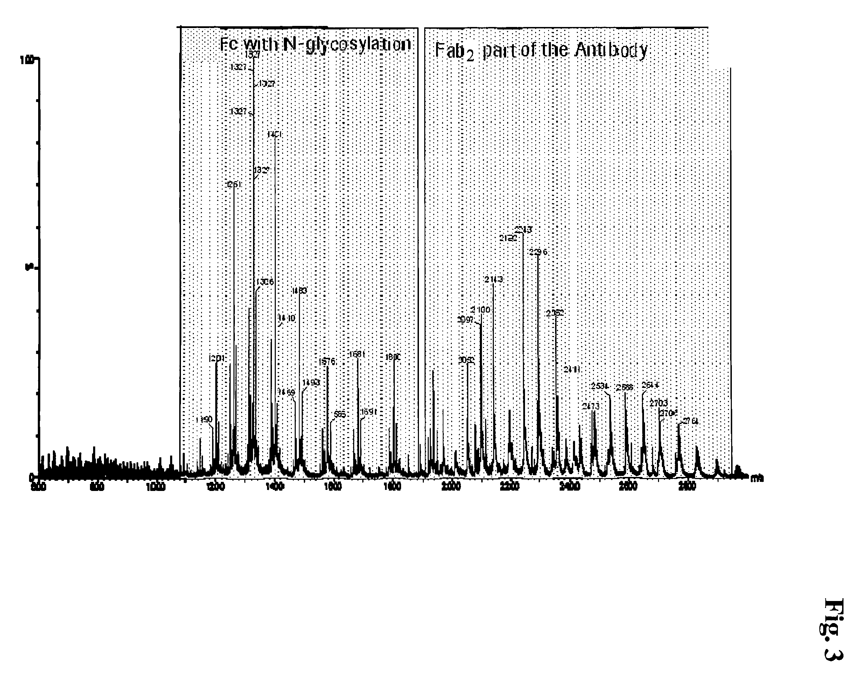

In the offline method the Fc glycosylation pattern determination comprises a SEC (size exclusion chromatography) desalting step and direct infusion of the sample into the mass spectrometer. This method is extremely fast, capable for high throughput and benefits from the high resolution, accuracy and sensitivity of oligosaccharide analysis at the level of HC Fc-fragment of immunoglobulins. The glycosylated HC Fc-fragment appears in the mass spectrum in the m/z range of 800 to 2000 (see e.g. Figure 2) whereas the Fab₂-fragment appears due to its double weight in the range from 1900 to 3000 m/z (see e.g. Figure 3). The two fragments of the immunoglobulin can therefore be separated by mass spectrometry which is an effect that facilitates data interpretation.

Thus, herein reported is a method for the determination of the glycosylation pattern of a human or humanized immunoglobulin of the subclass IgG1 or IgG2 or IgG4 or of a murine immunoglobulin of the subclass IgG2a or IgG2b or IgG3 or a variant thereof comprising the following steps:
a) digesting the immunoglobulin by treatment with the enzyme IdeS,
b) desalting the enzymatically cleaved fragments of the immunoglobulin obtained by the enzymatic digestion by a size exclusion chromatography,
c) subjecting the separated fragments of the immunoglobulin obtained in step b) to a mass spectrometric analysis, and
d) determining the glycosylation pattern of the immunoglobulin from the mass spectrometric data obtained in step c).

The methods according to the invention comprises as step a) the following step:
a) digesting the immunoglobulin by treatment with the enzyme IdeS and with the enzyme carboxypeptidase B.

The immunoglobulin is cleaved by the enzyme IdeS in order to obtain the HC Fc-fragment and the Fab₂-fragment and in the same step the C-terminal lysine of the immunoglobulin heavy chain is removed in order to reduce the heterogeneity of the immunoglobulin, i.e. to obtain a more homogeneous sample without influencing the glycosylation pattern.

The term "Fab₂-fragment" as used within this application denotes the N-terminal part of an immunoglobulin that is obtained by an enzymatic cleavage with the enzyme IdeS. The term "HC Fc-fragment" as used within this application denotes the C-terminal parts of the immunoglobulin heavy chains that are obtained by an enzymatic cleavage with the enzyme IdeS. This fragment comprises two not covalently associated polypeptides, i.e. the C-terminal fragment of each of the heavy chains. As the enzyme IdeS cleaves the immunoglobulin at a position different from the enzymes papain and pepsin and at only a single site of the immunoglobulin only two but well defined fragments of an immunoglobulin are obtained.

The method comprises a step a1) after step a) and before step b) which is:
a1) treating the enzymatically cleaved fragments of the immunoglobulin obtained by the enzymatic digestion of step a) with formic acid.

The Fc-fragment obtained from the immunoglobulin by the cleavage with the enzyme IdeS, which comprises polypeptides not covalently bound to each other, is separated in two pFc-fragments. Ther term "pFc-fragment" as used within this application denotes the single C-terminal polypeptide obtained from an immunoglobulin heavy chain after enzymatic cleavage with the enzyme IdeS.

The step a1) of the method according to the invention is:
a1) treating the enzymatically cleaved fragments of the immunoglobulin obtained by the enzymatic digestion of step a) with formic acid and a reducing agent.

The Fab₂-fragment obtained after the enzymatic cleavage of the immunoglobulin with the enzyme IdeS is further separated in two Fab-fragments by reduction of the connecting disulfide bonds. The formic acid is added at the same time as the addition of the reducing agent. Said reducing agent is a TCEP solution. The formic acid and the TCEP solution are both added prior to the incubation, so both components are present during a single step.

The method according to the invention comprises the incubation of a sample containing an immunoglobulin from which the glycosylation pattern has to be determined with different enzymes and agents. These compounds and agents are used to convert the immunoglobulin contained in the sample into defined fragments. In one embodiment of the method the IgG-specific cysteine protease IdeS is the IdeS obtained from Streptococcus pyogenes or Treponema denticola. In a further preferred embodiment the IgG-specific cysteine protease has the amino acid sequence SEQ ID NO: 1. The enzymatic cleavage with the IgG-specific cysteine protease takes place in one embodiment in a pH range between pH 5.5 and pH 8.5. In one embodiment the enzymatic cleavage is in the pH range of from pH 7.0 to pH 8.0. It was also found that the molar ratio of the IgG-specific cysteine protease to the immunoglobulin molecule should be between 1:25 and 1:2500, in another embodiment between 1:25 and 1:100.

In one embodiment of the method according to the invention the eluate of the reversed phase high performance liquid chromatography or the size exclusion chromatography is directly infused into the mass spectrometer.

If the glycosylation pattern of the Fab-fragment of an immunoglobulin has to be determined the method comprises the following steps:
a) digesting the immunoglobulin by treatment with the enzyme IdeS,
b) treating the enzymatically cleaved immunoglobulin of step a) with formic acid and a reducing agent,
c) separating the obtained fragments of said immunoglobulin by reversed phase high performance liquid chromatography and/or desalting the obtained fragments by size exclusion chromatography,
d) subjecting the separated fragments of the immunoglobulin obtained in step c) to a mass spectrometric analysis by direct infusion into a mass spectrometer, and
e) determining the glycosylation pattern of the immunoglobulin from the mass spectrometric data obtained in step d).

Herein is reported a method for the determination of the glycosylation pattern of a recombinantly produced human or humanized immunoglobulin of the subclass IgG1 or IgG2 or IgG4 or of a murine immunoglobulin of the subclass IgG2a or IgG2b or IgG3 comprising the following steps:
a) providing a sample of the recombinantly produced immunoglobulin,
b) digesting the immunoglobulin by treatment with the enzyme IdeS,
c) treating the enzymatically cleaved immunoglobulin of step b) with formic acid and a reducing agent,
d) separating the obtained fragments of said immunoglobulin by reversed phase high performance liquid chromatography and/or desalting the obtained fragments by size exclusion chromatography,
e) subjecting the separated fragments of the immunoglobulin obtained in step d) to a mass spectrometric analysis by direct infusion into a mass spectrometer, and
f) determining the glycosylation pattern of the immunoglobulin from the mass spectrometric data obtained in step e).

Herein is reported a method for the production of a Fab₂-fragment or a Fab-fragment of a human or humanized immunoglobulin of the subclass IgG1 or IgG2 or IgG4 or of a murine immunoglobulin of the subclass IgG2a or IgG2b or IgG3 comprising the following steps:
a) providing an immunoglobulin, from which the Fab₂-fragment or the Fab-fragment is to be obtained,
b) cleaving said immunoglobulin by digestion with the enzyme IdeS,
c) if the Fab-fragment is to be produced treating said enzymatically cleaved immunoglobulin of step b) with formic acid and a reducing agent,
d) producing said Fab₂-fragment or said Fab-fragment by chromatography with a protein A chromatography or a size exclusion resin.

For the purification of recombinantly produced heterologous immunoglobulins often a combination of different column chromatography steps is employed. In one embodiment a protein A affinity chromatography is followed by one or two additional chromatographic separation steps, e.g. ion exchange chromatographic steps. The final purification step is a so called "polishing step" for the removal of trace impurities and contaminants like aggregated immunoglobulins, residual HCP (host cell protein), DNA (host cell nucleic acid), viruses, and/or endotoxins. For this polishing step often an anion exchange chromatography material in a flow-through mode is used.

Different methods are well established and widespread used for protein recovery and purification, such as affinity chromatography with microbial proteins (e.g. protein A or protein G affinity chromatography), ion exchange chromatography (e.g. cation exchange (carboxymethyl resins), anion exchange (amino ethyl resins) and mixed-mode exchange), thiophilic adsorption (e.g. with beta-mercaptoethanol and other SH ligands), hydrophobic interaction or aromatic adsorption chromatography (e.g. with phenyl-sepharose, aza-arenophilic resins, or m-aminophenylboronic acid), metal chelate affinity chromatography (e.g. with Ni(II)- and Cu(II)-affinity material), size exclusion chromatography, and electrophoretical methods (such as gel electrophoresis, capillary electrophoresis) (Vijayalakshmi, M.A., Appl. Biochem. Biotech. 75 (1998) 93-102).

In one embodiment the method comprises cultivating a eukaryotic cell comprising a nucleic acid encoding the immunoglobulin under conditions suitable for the expression of the heterologous immunoglobulin. The term "under conditions suitable for the expression of the immunoglobulin" denotes conditions which are used for the cultivation of a mammalian cell expressing an immunoglobulin and which are known to or can easily be determined by a person skilled in the art. It is also known to a person skilled in the art that these conditions may vary depending on the type of mammalian cell cultivated and on the type of immunoglobulin expressed. In general the mammalian cell is cultivated at a temperature, e.g. between 20 °C and 40 °C, and for a period of time sufficient to allow effective protein production of the immunoglobulin, e.g. for 4 to 28 days.

Herein is reported a method for the monitoring of the glycosylation patter of a recombinantly produced immunoglobulin by using a method according to the invention.

Still further reported herein is a method for producing a protein comprising the step of:
a) enzymatically cleaving a protein-immunoglobulin fusion protein with the enzyme IdeS and thereby producing the protein.

The following examples, sequence listing and figures are provided to aid the understanding of the present invention, the true scope of which is set forth in the appended claims.

### Description of the Sequence Listing

| | |
|---|---|
| **SEQ ID NO: 1** | Amino acid sequence of IdeS from Streptococcus pyogenes. |
| **SEQ ID NO: 2** | Murine IgG2a immunoglobulin sequence starting at C_{H}1 and ending with C_{H}2. |
| **SEQ ID NO: 3** | Murine IgG3 immunoglobulin sequence starting at C_{H}1 and ending with C_{H}2. |
| **SEQ ID NO: 4** | Human IgG1 constant region. |
| **SEQ ID NO: 5** | Human IgG4 constant region. |

**Description of the Figures**
- **Figure 1**: Schematic representation of the IdeS digestion of IgG1 immunoglobulins.
- **Figure 2**: Mass spectrum of IdeS cleaved human IgG1 analyzed by SEC and direct infusion into mass spectrometer. The spectrum shows only the glycosylated Fc fragment.
- **Figure 3**: Mass spectrum of IdeS cleaved human IgG1 analyzed by SEC and direct infusion into mass spectrometer. The spectrum shows the glycosylated Fc-fragment and the Fab₂-fragment.
- **Figure 4**: Chromatogram of IdeS digested and reduced murine IgG3 immunoglobulin.
- **Figure 5**: Zoom mass spectrum of heavy chain Fab-fragment with O-glycosylation in the hinge region of murine IgG3 immunoglobulin.
- **Figure 6**: Zoom mass spectrum of heavy chain Fc-fragment with N-glycosylation of murine IgG3 immunoglobulin.
- **Figure 7**: Deconvoluted mass spectrum of the O-Glycosylated tryptic peptide (IPKPSTPPGSSCPPGNILGGPSVFIFPPKPK (HC = heavy chain, amino acid (aa) 217-247; S and T: possible O-glycosylation sites) as obtained from LC-MS.
- **Figure 8**: Chromatogram of IdeS digested humanized IgG4 immunoglobulin.
- **Figure 9**: Zoom mass spectrum of heavy chain Fc-fragment with N-glycosylation of humanized IgG4 immunoglobulin.
- **Figure 10**: Chromatogram of IdeS digested humanized IgG1 immunoglobulin.
- **Figure 11**: Zoom mass spectrum of heavy chain Fc-fragment with N-glycosylation of humanized IgG1 immunoglobulin.
- **Figure 12**: Overlay of chromatograms of IdeS digested humanized IgG1 immunoglobulin taken from the supernatant of a cultivation and a protein A purified sample.

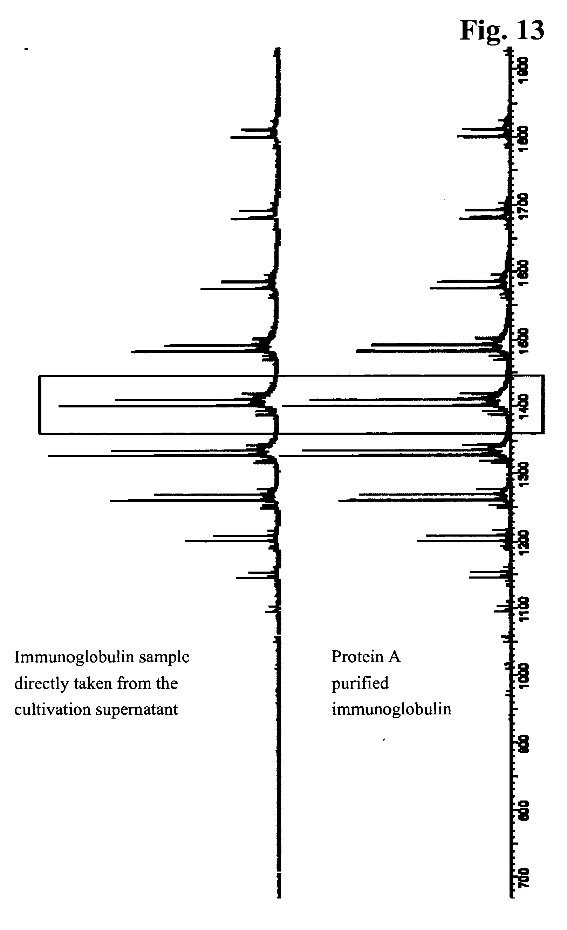

- **Figure 13**: Overlay of mass spectra of IdeS digested humanized IgG1 immunoglobulin taken from the supernatant of a cultivation and a protein A purified sample (retention time: 16.8 minutes).

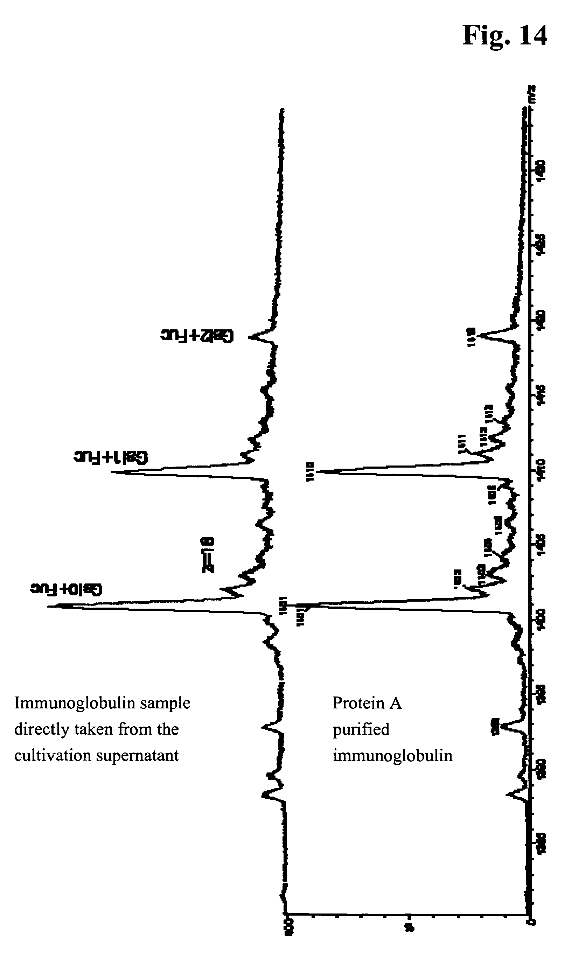

- **Figure 14**: Zoom overlay of mass spectra of IdeS digested humanized IgG1 immunoglobulin taken from the supernatant of a cultivation and a protein A purified sample (retention time: 16.8 minutes).
- **Figure 15**: Analytical size exclusion chromatogram overlay of the complete immunoglobulin, the Fab₂-fragment and the standard.

### Example 1

### Determination of the antibody concentration:

The antibody concentration was determined by means of an absorption measurement at 280 nm on a spectral photometer of the type UVIKON XL (Goebel Company). The extinction coefficient of the antibody that was used was 1.55 ml/(mg*cm) and was calculated according to the method of Pace, C.N., et al., (Protein Sci. 4 (1995) 2411-2423).

### Example 2

### General Method A (for IgG1, IgG3)

### IdeS digestion for analysis of the glycosylation pattern in the Fc-fragment:

100 µg (0.66 nmol) immunoglobulin are diluted to a final concentration of 1 mg/ml in 50 mM TRIS/HCl buffer pH 8.0 and 2 µl (c = 1 mg/ml, 0.06 nmol) of Immunoglobulin degrading enzyme (IdeS, MW 345890 Da) are added to give an enzyme to immunoglobulin ratio of 1:50 by weight. The solution is incubated for 2 to 5 h at 37 °C depending on the immunoglobulin used. For analysis with size exclusion chromatography and direct infusion into mass spectrometer the enzyme activity is stopped by addition of an equal volume of 1 % formic acid to the solution.

### Reduction of IdeS digested immunoglobulin for additional analysis of glycosylation pattern in the Fab-fragment:

For reduction half of the sample is diluted with 64 µl of 100 mM potassium phosphate buffer pH 7.0 to give a final volume of 115 µl. Then, 60 µl of 0.5 M TCEP (tris(2-carboxyethyl) phosphine, Pierce) dissolved in a volume of 4 M guanidine hydrochloride and 50 ml of 8 M guanidine hydrochloride are added. Afterwards the sample is incubated for 30 minutes at 37 °C. The reaction is stopped by addition of 5 µl of 20 % (v/v) formic acid.

### Example 3

### General Method B (for IgG1, IgG3, IgG4)

### Combined IdeS and CpB digest:

100 µg (0.66 nmol) immunoglobulin are diluted to a final concentration of 1 mg/ml in 50 mM TRIS/HCl buffer pH 8.0 and 2 µl (c = 1 mg/ml, 0.06 nmol) of Immunoglobulin degrading enzyme (IdeS, MW 345890 Da) are added to give an enzyme to immunoglobulin ratio of 1:50 by weight. The solution is incubated for 2 h to 5 h at 37 °C depending on the immunoglobulin used. 1 µl (1 mg/ml) of Carboxypeptidase B (CpB, Roche Diagnostics GmbH, Mannheim, Germany) is added to the solution 30 minutes before the end of the IdeS incubation time to give an enzyme to immunoglobulin ratio of 1:25 by weight.

### Example 4

### General Method C (for IgG with complex glycosylation pattern)

### Combined IdeS and EndoH digest:

25 µg (0.17 nmol) immunoglobulin are diluted to a final concentration of 0.5 mg/ml in sodium phosphate buffer at pH 6.0 and 2.5 µl (c = 2.5 U/500 µl) of Endoglycosidase H (Roche Diagnostics GmbH, Mannheim, Germany) are added and incubated for 18 h at 37 °C to cleave the oligosaccharide structures. Subsequently, the pH is adjusted to 8.0 by adding 25 µl of 0.1 M TRIS/HCl buffer pH 8.0. Cleavage of the immunoglobulin is achieved by adding 0.5 µl of (c = 1 mg/ml, 0.02 nmol) of Immunoglobulin degrading enzyme (IdeS, MW 345890 Da) and incubation for 2 h at 37°C.

### Example 5

### General Method D (for cultivation supernatants)

### Combined IdeS and CpB digest:

50 µg (0.33 nmol) of a cultivation supernatant of a cultivation of an immunoglobulin expressing eukaryotic cell are centrifuged for 3 minutes at 10,800 rcf (relative centrifugal force) and diluted to a final concentration of 0.7 mg/ml in 50 mM TRIS/HCl buffer pH 8.0. 1 µl (c = 1 mg/ml, 0.06 nmol) of Immunoglobulin degrading enzyme (IdeS, MW 345890 Da) is added to give an enzyme to immunoglobulin ratio of 1:50 by weight. The solution is incubated for 2 to 5 h at 37 °C depending on the immunoglobulin used. 1 µl (1 mg/ml) of Carboxypeptidase B (CpB, Roche Diagnostics GmbH, Mannheim, Germany) is added to the solution 30 minutes before the end of the IdeS incubation time to give an enzyme to immunoglobulin ratio of 1:25 by weight.

### Example 6

### RP-HPLC-MS method

The LC-MS is performed on an Agilent Cap LC1100 coupled to QTOF II (Micromass/Waters). The chromatographic separation is performed on a Phenomenex Jupiter C18 column (5 µm particle size, 300 Å pore size, 1 x 250 mm column). Eluent A is 0.5 % formic acid, eluent B is 70 % Isopropanol, 20 % acetonitrile, 9.5 % Water and 0.5 % formic acid. The flow is 40 µl/min, the separation is performed at 75 °C and 2 µg (10 µl) of immunoglobulin obtained with a method according to one of the Examples 2 to 5 are injected onto the column. Following gradient is applied:

| **Time (min)** | **% B** |
|---|---|
| 0 | 20 |
| 7 | 20 |
| 9 | 25 |
| 29 | 50 |
| 32 | 100 |
| 37 | 100 |
| 38 | 20 |
| 50 | 20 |

During the first 7 minutes the eluate is directed into the waste to prevent the mass spectrometer ion source from salt contamination. UV signal at 280 nm (reference 360 nm) is recorded. MS spectra are acquired using a capillary voltage of 2,700 V, a cone voltage of 30 V in a mass range from 600 to 2000 m/z in positive ion mode using a desolvation temperature of 120 °C and a source temperature of 80 °C. MS data are acquired from 7 to 50 minutes.

### Example 7

### Glycoanalysis of IdeS cleaved antibody by direct infusion

### Size exclusion chromatography:

45 µg (90 µl) of IdeS cleaved immunoglobulin obtained with a method according to one of the Examples 2 to 5 are injected onto a Sephadex G25 self packed ECO SR column (5 x 250 mm) (KronLab) equilibrated with 2 % formic acid, 40 % acetonitrile at a flow rate of 0.5 ml/min for 30 minutes. The protein is desalted using an 8 minute isocratic elution with 2 % formic acid, 40 % acetonitrile at a flow rate of 1 ml/min. The elution of the desalted protein is recorded by UV (280 nm) and sample is collected via fraction collector into microtiter plates. The microtiter plates can be inserted into a TriversaNanoMate (Advion) system and MS spectra are recorded automatically or sample can be pipetted manually into a metal coated glass needles (Proxeon Biosystems Nano ESI-needles, cat# ES387) and sprayed into the mass spectrometer.

### MS parameter for direct infusion on a QTOF II instrument (Micromass/Waters):

MS spectra are acquired using a capillary voltage of 800 V, a cone voltage of 33 V in a mass range from 600 to 2000 m/z (glycosylated Fc-fragment) in positive ion mode using a desolvation temperature of 120 °C and a source temperature of 80 °C. MS data are acquired for approx. 2 minutes.

### Example 8

### Glycoanalysis of a murine IgG3 immunoglobulin

The method according to the invention has been exemplified in this Example with a murine IgG3 immunoglobulin. This immunoglobulin has two glycosylation sites, one in the Fab-fragment and one in the Fc-fragment.

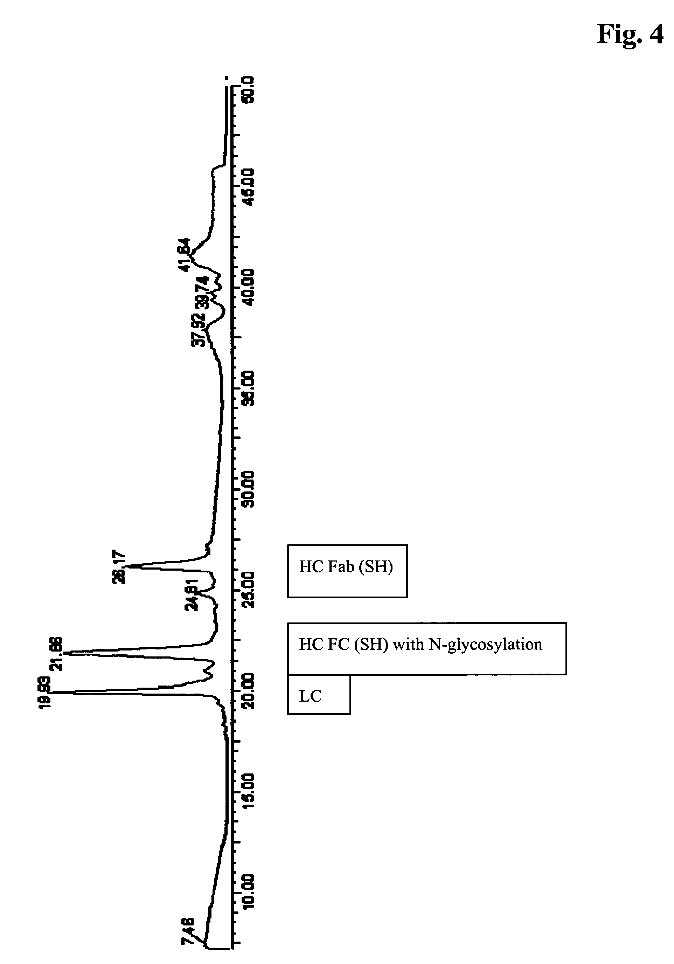

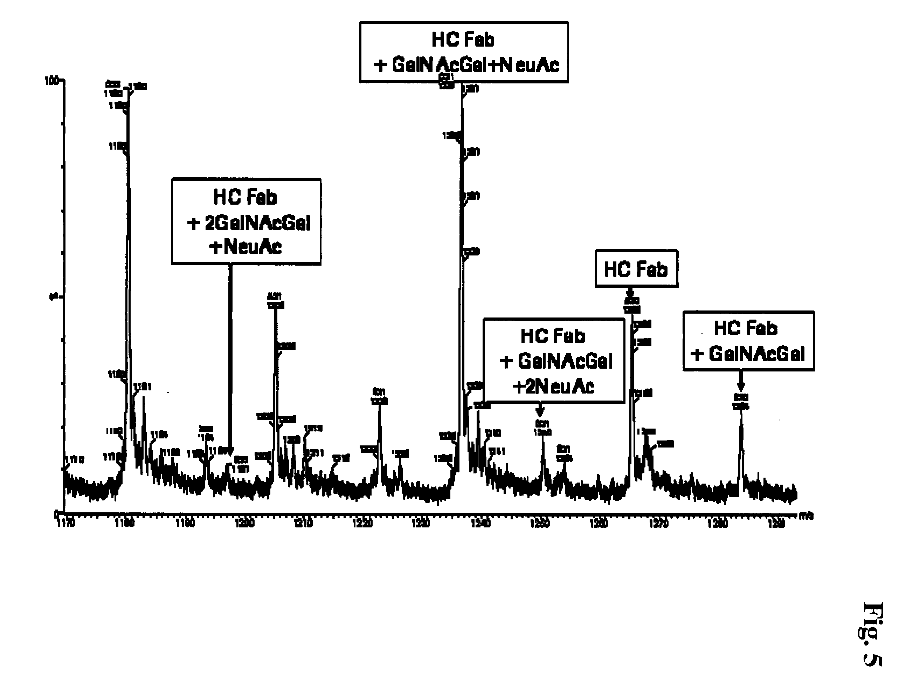

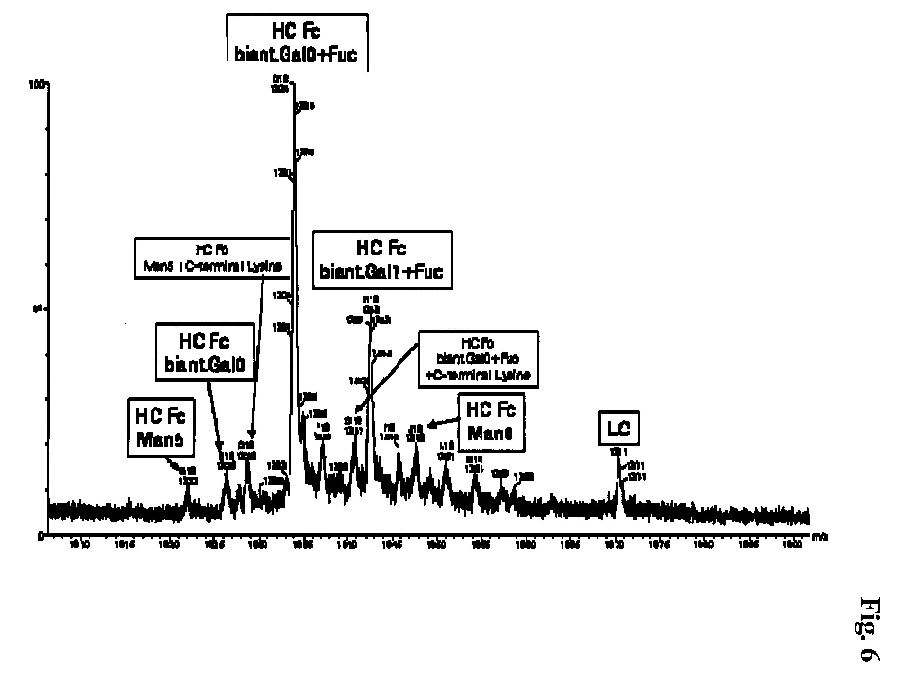

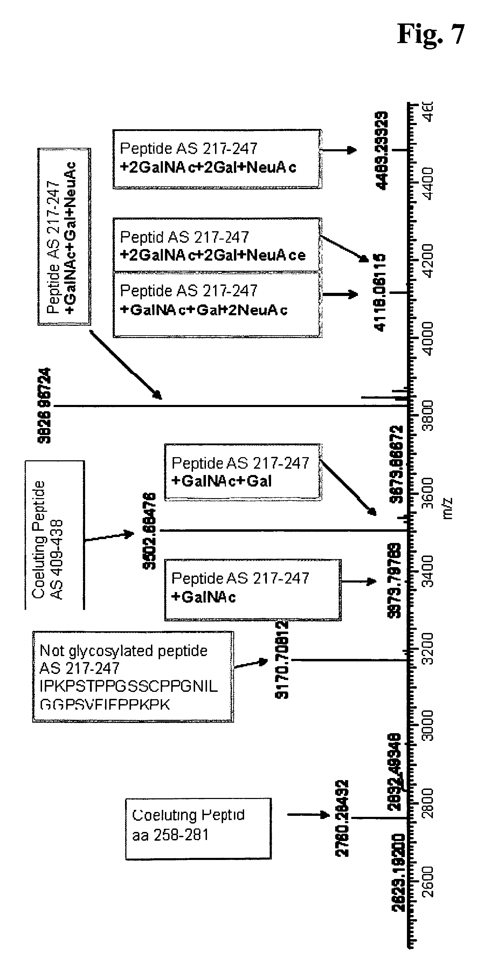

The chromatogram of an IdeS digested and reduced murine IgG3 immunoglobulin is shown in Figure 4. A zoom of the mass spectrum of the heavy chain Fab-fragment with the O-glycosylation in the hinge region is shown in Figure 5, whereas a zoom of the mass spectrum of the heavy chain Fc-fragment with the N-glycosylation site is shown in Figure 6. In Figure 7 a deconvoluted mass spectrum of the O-glycosylated tryptic peptide is shown with the glycosylation pattern.

### Example 9

### Glycoanalysis of a humanized IgG4 immunoglobulin

The method according to the invention has been exemplified in this Example with a humanized immunoglobulin comprising a human IgG4 immunoglobulin constant region. This immunoglobulin has one N-glycosylation site in the Fc-fragment.

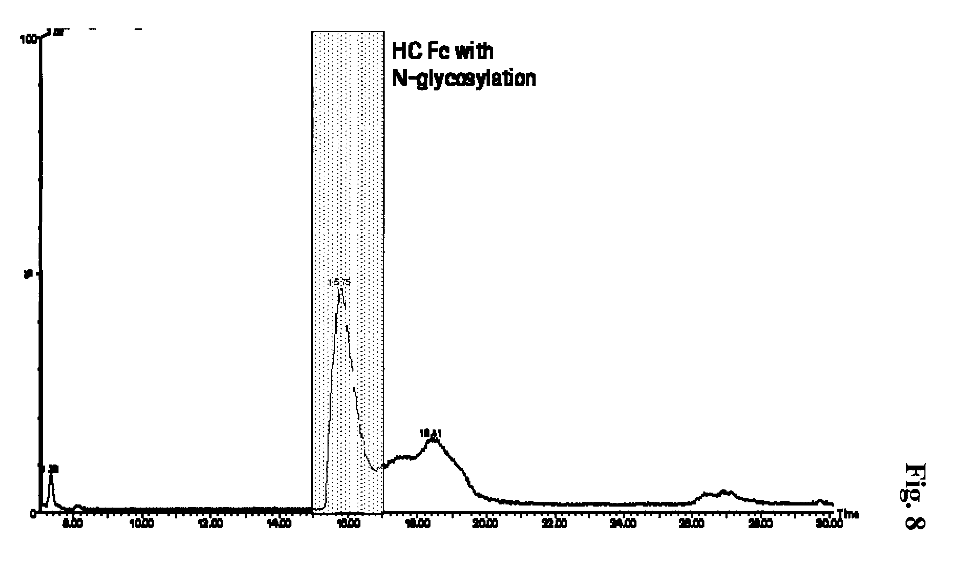

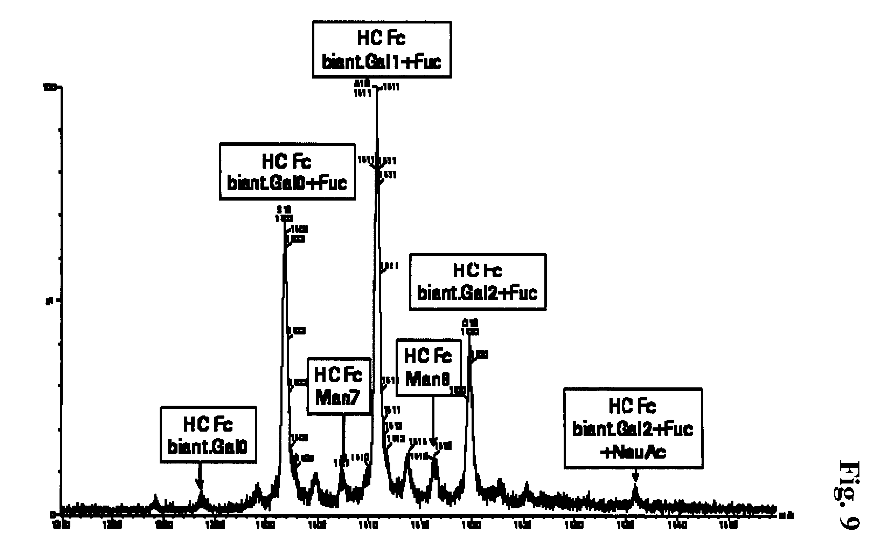

The chromatogram of the IdeS digested humanized IgG4 immunoglobulin is shown in Figure 8. A zoom of the mass spectrum of the heavy chain Fc-fragment with the N-glycosylation site is shown in Figure 9.

### Example 10

### Glycoanalysis of a humanized IgG1 immunoglobulin

The method according to the invention has been exemplified in this Example with a humanized immunoglobulin comprising a human IgG1 immunoglobulin constant region. This immunoglobulin has one N-glycosylation site in the Fc-fragment.

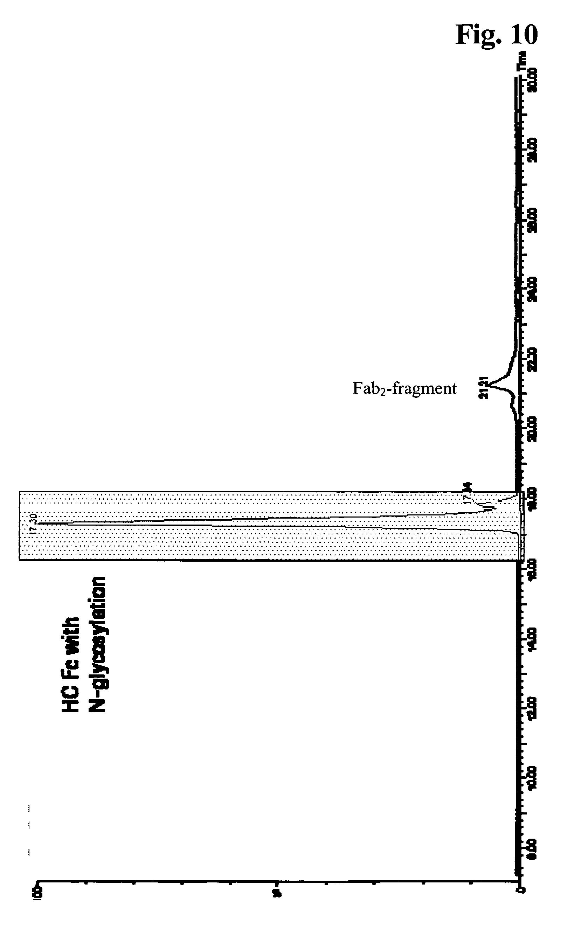

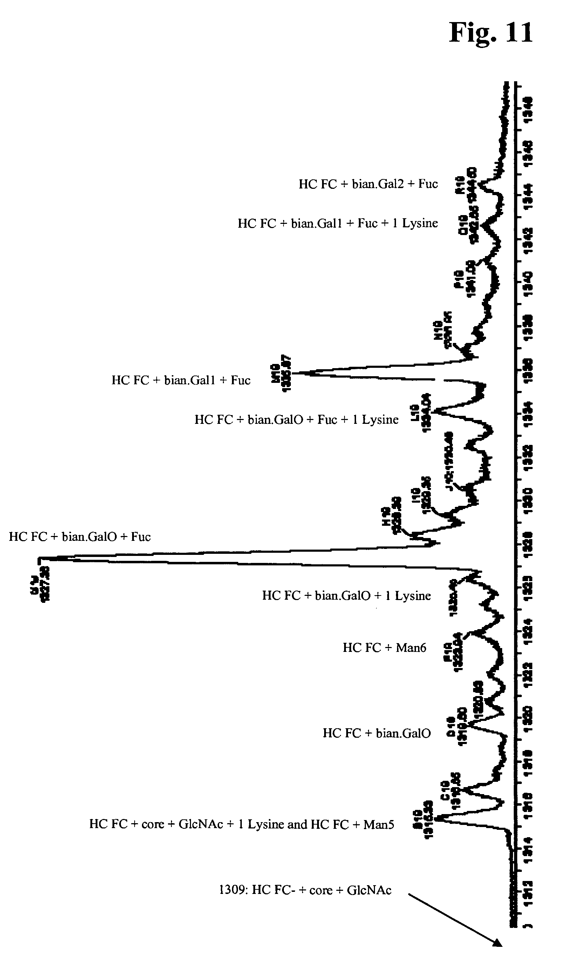

The chromatogram of the IdeS digested humanized IgG1 immunoglobulin is shown in Figure 10. A zoom of the mass spectrum of the heavy chain Fc-fragment with the N-glycosylation site is shown in Figure 11.

### Example 11

### Glycoanalysis of a humanized IgG1 immunoglobulin from a cultivation supernatant

The method according to the invention has been exemplified in this Example with a humanized immunoglobulin comprising a human IgG1 immunoglobulin constant region whereby the sample for analysis was obtained directly from culture supernatant of a cultivation without further purification. This immunoglobulin has one N-glycosylation site in the Fc-fragment. This Example shows that it is possible to use the method according to the invention as an online tool for monitoring the glycosylation during the cultivation of a eukaryotic cell.

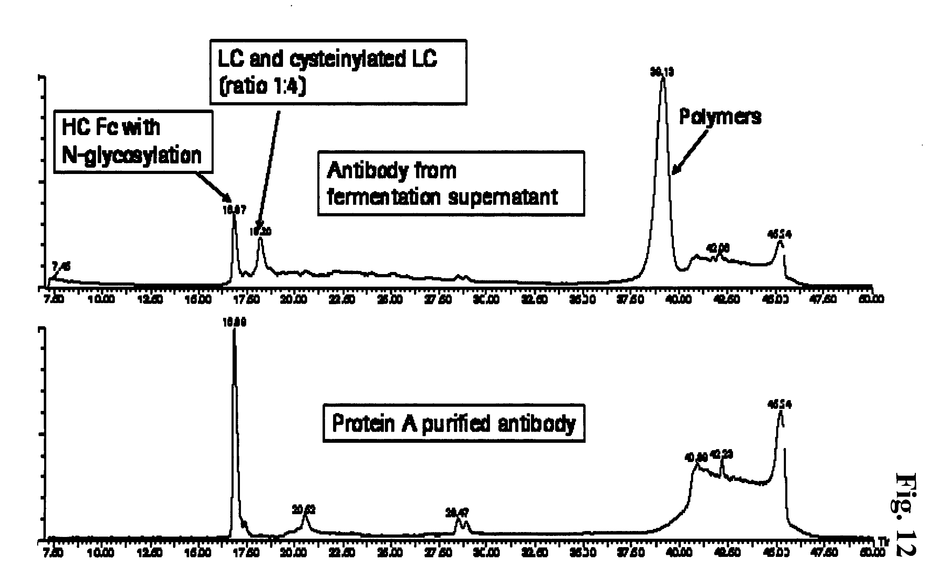

The chromatogram of the IdeS digested sample from the culture supernatant in comparison with a sample purified with a protein A chromatography is shown in Figure 12. It can be seen that the heavy chain Fc-fragment is eluted at the same point of the chromatogram. The chromatogram of the protein A purified sample does not contain the light chain of the immunoglobulin as the light chain is removed during protein A chromatography.

### Example 12

### Production of Fab₂-fragment of a humanized IgG1

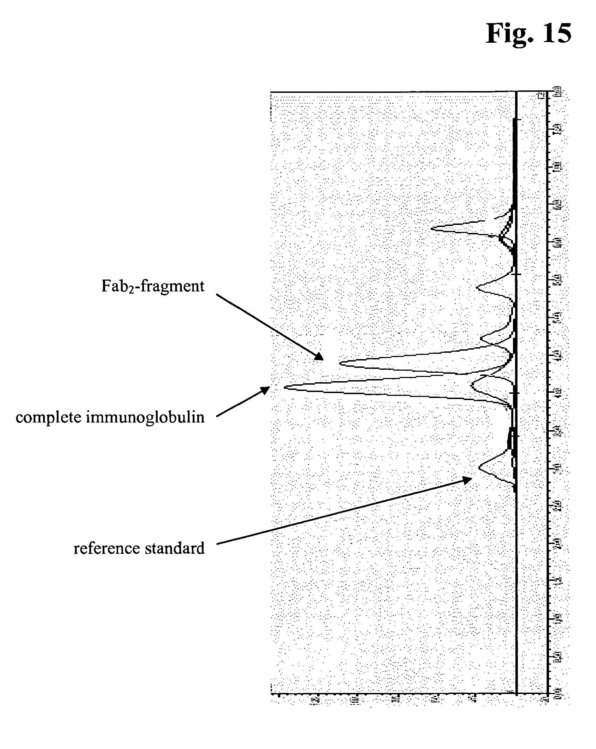

10 mg immunoglobulin are diluted to a final concentration of 1 mg/ml in 50 mM TRIS/HCl buffer pH 8.0 and 18 µl (c = 11.3 mg/ml) of Immunoglobulin degrading enzyme (IdeS, MW 345890 Da) are added to give an enzyme to immunoglobulin ratio of 1:50 by weight. The solution is incubated for 0.5 to 2 h at 37 °C with stirring. Directly after the incubation with IdeS a protein A column chromatography was performed using a protein A HP high trap column. Buffer A was phosphate buffered saline at pH 7.4, buffer B was 100 mM sodium citrate buffer pH 2.8 with a flow rate of 1 ml/min. The Fab₂-fragment is obtained from the flow through of the column whereas the Fc-fragment is obtained by elution with buffer B. The obtained fractions have a purity of from 84 % to 95 % when determined by size exclusion chromatography. A further purification step using a size exclusion chromatography with a Superdex 75 HighLoad 16/60 column with a volume of 120 ml can be performed. As buffer 20 mM sodium phosphate buffer with 140 mM sodium chloride, pH 6.0 was used with a flow of 1 ml/min. As can be seen from Figure 15 the original complete antibody has been converted to its Fab₂-fragment.

## Claims

1. Method for the determination of the glycosylation pattern of a human or humanized immunoglobulin of the subclass IgG1 or IgG2 or IgG4 or a Fab₂-fragment thereof comprising the following steps:
a) cleaving said immunoglobulin into fragments by enzymatic digestion with the enzyme IdeS and treating said immunoglobulin with carboxypeptidase B,
b) treating said enzymatically cleaved immunoglobulin with formic acid and TCEP,
c) separating the fragments of said immunoglobulin obtained by the enzymatic digestion by reversed phase high performance liquid chromatography,
d) subjecting the separated fragments of said immunoglobulin obtained in step c) to a mass spectrometric analysis wherein the mass spectrometric analysis is performed in the absence of trifluoro acetic acid, and
e) determining the glycosylation pattern of said immunoglobulin from the mass spectrometric data obtained in step d).

2. Method according to claim 1, **characterized in that** said separating step is:
- desalting the obtained fragments of said immunoglobulin by a size exclusion chromatography.

3. Method according to any one of the preceding claims, **characterized in that** said cleaving is performed in a pH range between pH 5.5 and pH 8.5.

4. Method according to any one of the preceding claims, **characterized in that** the cleaving is performed for two hours.

5. Method according to any one of the preceding claims, **characterized in that** the molar ratio of IdeS to the immunoglobulin molecule is between 1:25 and 1:2500.

## Patentansprüche

1. Verfahren zur Bestimmung des Glycosylierungsmusters eines humanen oder humanisierten Immunglobulins der Unterklasse IgG1 oder IgG2 oder IgG4 oder eines Fab₂-Fragments davon, das folgende Schritte umfasst:
a) Spalten des Immunglobulins in Fragmente durch enzymatischen Verdau mit dem Enzym IdeS und Behandeln des Immunglobulins mit Carboxypeptidase B,
b) Behandeln des enzymatisch gespaltenen Immunglobulins mit Ameisensäure und TCEP,
c) Trennen der durch den enzymatischen Verdau erhaltenen Fragmente des Immunglobulins durch Umkehrphasen-Hochleistungsflüssigkeitschromatographie,
d) massenspektrometrische Analyse der in Schritt c) erhaltenen getrennten Fragmente des Immunglobulins, wobei die massenspektrometrische Analyse in Abwesenheit von Trifluoressigsäure durchgeführt wird, und
e) Bestimmen des Glycosylierungsmusters des Immunglobulins aus den in Schritt d) erhaltenen massenspektrometrischen Daten.

2. Verfahren nach Anspruch 1, **dadurch gekennzeichnet, dass** es sich bei dem Trennschritt um
- Entsalzen der erhaltenen Fragmente des Immunglobulins durch Größenausschlusschromatographie handelt.

3. Verfahren nach einem der vorhergehenden Ansprüche, **dadurch gekennzeichnet, dass** die Spaltung in einem pH-Bereich zwischen pH 5,5 und pH 8,5 durchgeführt wird.

4. Verfahren nach einem der vorhergehenden Ansprüche, **dadurch gekennzeichnet, dass** die Spaltung über einen Zeitraum von zwei Stunden durchgeführt wird.

5. Verfahren nach einem der vorhergehenden Ansprüche, **dadurch gekennzeichnet, dass** das Molverhältnis von IdeS zu dem Immunglobulin-Molekül zwischen 1:25 und 1:2500 liegt.

## Revendications

1. Procédé pour la détermination du motif de glycosylation d'une immunoglobuline humaine ou humanisée de la sous-classe IgG1 ou IgG2 ou IgG4 ou d'un fragment Fab₂, comprenant les étapes suivantes :
a) le clivage de ladite immunoglobuline en fragments par digestion enzymatique avec l'enzyme IdeS et traitement de ladite immunoglobuline avec de la carboxypeptidase B ;
b) le traitement de ladite immunoglobuline clivée enzymatiquement avec de l'acide formique et du TCEP,
c) la séparation des fragments de ladite immunoglobuline obtenus par la digestion enzymatique par chromatographie en phase liquide à hautes performances à phase inversée ;
d) l'analyse par spectrométrie de masse des fragments séparés de ladite immunoglobuline obtenus dans l'étape c), ladite analyse par spectrométrie de masse étant effectuée en l'absence d'acide trifluoroacétique, et
e) la détermination du motif de glycosylation de ladite immunoglobuline d'après les résultats de spectrométrie de masse obtenus dans l'étape d).

2. Procédé suivant la revendication 1, **caractérisé en ce que** ladite étape de séparation est :
- l'élimination des sels des fragments obtenus de ladite immunoglobuline par une chromatographie d'exclusion dimensionnelle.

3. Procédé suivant l'une quelconque des revendications précédentes, **caractérisé en ce que** ledit clivage est effectué à un pH dans la plage entre pH 5,5 et pH 8,5.

4. Procédé suivant l'une quelconque des revendications précédentes, **caractérisé en ce que** le clivage est effectué pendant 2 heures.

5. Procédé suivant l'une quelconque des revendications précédentes, **caractérisé en ce que** le rapport molaire de la IdeS à la molécule d'immunoglobuline est compris entre 1:25 et 1:2500.
